# EUROPEAN PATENT SPECIFICATION

(11) **EP 0 942 745 B1**
(45) Date of publication and mention of the grant of the patent: **25.09.2002**
(21) Application number: 97934835.6
(22) Date of filing: 24.07.1997
(51) Int. Cl.: A61K 38/47, A61P 7/10

(54) **USE OF HYALURONIDASE**
VERWENDUNG VON HYALURONIDASE
UTILISATION DE HYALURONIDASE

(30) Priority: 26.08.1996 SE 9603082
(43) Date of publication of application: 22.09.1999
(73) Proprietor: Tufveson, Gunnar, 756 47 Uppsala (SE); Hällgren, Roger, 740 22 Balinge (SE); Johnsson, Cecilia, 752 41 Uppsala (SE); Wahlberg, Jan, 754 49 Uppsala (SE)
(72) Inventor: Tufveson, Gunnar, 756 47 Uppsala (SE); Hällgren, Roger, 740 22 Balinge (SE); Johnsson, Cecilia, 752 41 Uppsala (SE); Wahlberg, Jan, 754 49 Uppsala (SE)
(74) Representative: Bergvall-Eftring, Stina Lena
(86) International application number: SE9701313
(87) International publication number: WO98008538

(56) References cited:
- J. CLIN. INVEST., Volume 88, November 1991, ANDERS WALDENSTROM et al., "Accumulation of Hyaluronan and Tissue Edema in Experimental Myocardial Infarction", pages 1622-1628.
- ANGIOLOGY, Volume 41, No. 12, 1990, AKIRA TAIRA et al., "Active Drainage of Cardiac Lymph in Relation to Reduction in Size of Myocardial Infarction:- An Experimental Study", pages 1029-1036.
- TRANSPLANTATION, Volume 50, No. 2, August 1990, ALVIN F. WELLS et al., "The Localization of Hyaluronan in Normal and Rejected Human Kidneys", pages 240-243.
- MICROVASCULAR RESEARCH, Volume 30, 1985, KENNETH P. SUNNERGREN, "The Effects of Hyaluronidase on Interstitial Hydration, Plasma Protein Exclusion and Microvascular Permeability in the Isolated Perfused Rat Heart", pages 286-297.

## Description

The present invention relates to the use of hyaluronidase in the manufacturing of a pharmaceutical for the treatment of a mammal of human or animal origin suffering from a condition of interstitial edema associated with an increased local synthesis of hyaluronan in connection with organ grafting.

### BACKGROUND OF THE INVENTION

Hyaluronidase is an enzyme produced in e.g. mammals, leeches, and krill and which is extractable from a number of tissues. Hyaluronidase degrades a mucopolysaccharide called hyaluronan, also referred to as hyaluronic acid (Balazs EA et al, Biochem J 1986; 235: 903).

Hyaluronan is a linear polymer built up by the repeating disaccharide N-acetyl-D-glucosamine-D-glucuronic acid. It has a key role in the structure and organization of the extracellular matrix and is a major component of the extracellular matrix during embryogenesis (Toole, P.B. in Cellular Biology of Extracellular Matrix (ed. Hay, E.D.) 251-294 (Plenum Publishing Corp., New York, 1981) as well as whenever rapid tissue proliferation, regeneration, and repair occurs. It is produced by the mesenchymal cells (Comper WD, Laurent TC, Physiol Rev 1978; 58: 255-315).

Hyaluronan has several biological effects, that are in part related to its molecular size (West, D.C., Kumar, S. Exp. Cell. Res. 183, 179-196, 1989). The unique water-binding capacity of hyaluronan is well recognized and utilized for therapeutic purposes in e.g. eye surgery.

The content of hyaluronan in an organ has been shown to increase in different conditions of inflammation of that organ. Thus, an increased concentration of hyaluronan has been shown in tissue from different organs characterized by a condition of inflammatory-immunological injury such as alveolitis (Nettelbladt O et al, Am Rev Resp Dis 1989; 139: 759-762) and myocardial infarction (Waldenstrom et al, J Clin Invest 1991; 88(5): 1622-1628). Other examples of this type of conditions are the allograft rejection after a renal (Hällgren et al, J Exp Med 1990a; 171: 2063-2076; Wells et al, Transplantation 1990; 50: 240-243), small bowel (Wallander et al, Transplant Int 1993; 6: 133-137) or cardiac (Hällgren et al, J Clin Invest 1990b;85:668-673) transplantation; or a myocardial inflammation of viral origin (Waldenstrom et al, Eur J Clin Invest 1993; 23: 277-282).

Due to the pronounced water binding capacity of hyaluronan (Mason RM, Progr Clin Biol 1981; Res 54:87-112) an increased local production and accumulation of hyaluronan in the connective tissue of an organ leads to a local accumulation of liquid, with a risk of harmful interstitial tissue edema, which in turn may lead to an impairment of the function of that organ. It is clear that this accumulation of hyaluronan in e.g. an organ in a state of inflammation may result in very severe consequences. In the case of an organ transplantation the development of a transplantation edema presents a threat to the function of the organ graft due, for example, to an increase in the extracellular pressure and compression of the microvasculature or to an increased diffusion distance for oxygen. This evenually may lead to an irreversible rejection of the organ and in some cases even to a life threatening condition. Therefore it exists a great demand for a method of alleviating this state of risk. The object of the present invention is to provide a means of achieving this.

Hyaluronidase has previously, in the seventies, been used to reduce the effects of myocardial infarction (Maroko PR et al, Circulation 1972; 46(3): 430-7; Maclean D, et al, Science 1976; 194(4261): 199-200). The mode of action was however not understood. In an in vitro animal model, cardiac performance after ischemic injury was improved upon administration of hyaluronidase (Rovetto MJ, Circ-Res 1977; 41(3): 373-9; Fischer JH et al, Transplantation 1994; 58(6): 748-53).

Also, Waldenström et al. (J Clin Invest 1991; 88(5): 1622-1628), in view of the fact that hyaluronan is accumulated in the infarction area in the experimental myocardial infarction induced in rats, had speculated about a possible successful use of hyaluronidase in limiting cellular damage during ischemia, however without putting their theory into practice.

Hyaluronidase treatment also has been proposed by other workers as a means of treating myocardial infarction, possibly by stimulating the formation of a collateral blood flow, i.e. the formation of new blood vessels (Sleight P, Interventions during and after acute myocardial infarction. Postgrad. Med. J. 59, suppl 3: 80-8 (1983)). However, large randomized studies on human subjects in a state of acute infarction have not been able to corroborate this. In a study on 1488 subjects with acute myocardial infarction, no beneficial effect on lethality could be shown in the group treated with hyaluronidase compared to the placebo group (Julian DG, Simpson JM, Cadigan PJ, Petri MC et al. Cardiology 75(3): 177-183, (1988)). Another double-blind multi-center study on 851 subjects, suffering from myocardial infarction also was unable to show any significant difference between those treated with hyaluronidase and with placebo, respectively. (MILIS Study Group. Am J Cardiol 57(15): 1236-1243, (1986)).

Obviously, none of these large scale studies has been able to show any beneficial effect on myocardial infarction in human subjects from the treatment with hyaluronidase.

Active drainage of cardiac lymph in dogs using hyaluronidase has been successfully attempted in dogs (Taira A et al., Angiology (United States) Dec 1990, 41 (12) p1029-36, ISSN 003-3197, Journal Code: 4UA). Drainage of lymph and interstitial edema are only indirectly related to each other. In the invention proposed by us, it is presumed that the lymph drainage is normal.

Interstitial hyaluronan accumulation is well documented in several organs in response to ischemic/inflammatory injury. In transplanted organs hyaluronan is acculumated in the early post-operative period probably due to the ischemic damage induced by organ procurement and preservation. However, during development of acute rejection there is a progressive and much more pronounced interstitial hyaluronan accumulation, probably due to enhanced hyaluronan synthesis by interstitial cells stimulated by a number of inflammatory products related to immunological and/or inflammatory responses of the organ graft rejection.

The occurrence of interstitial edemas in connection with the grafting of an organ constitutes a severe problem in the field of transplantation surgery. Indeed; within e.g. the field of kidney transplantations, statistically as much as 25% of the grafts, i.e. 25 grafts out of 100, will swell to such a degree that the function will temporarily be lost. Moreover, in about 2-3% of the cases, the swelling is so severe that it causes disruption of the kidney, resulting in a massive haemorrhage.

The swelling of a transplanted organ thus will constitute a severe hazard to the subject undergoing the transplantation surgery. Clearly, there is an urgent need to find a remedy to this. However, in spite of this urgent need no one up to now had shown that there is a simple and reliable way of accomplishing this, as according to the present invention. The explanation to this may be found in the fact that from several reasons the man skilled in the art would not have deemed this to be an operable therpeutic method. Firstly, it was considered most improbable that the enzyme would reach the edematous tissue. Secondly, hyaluronidase has its optimum activity at an acidic pH, whereas the tissue pH is neutral, and thus to low an activity of the enzyme would be expected in the tissue. Finally, if the enzyme would nonetheless prove to be active, then there was a fear that it would cause degradation of hyaluronan not only in the edema but in th whole of the body.

Indeed, the discovery that it is possible to treat an interstitial edema associated with an increased local synthesis of hyaluronan in connection with organ grafting, which forms the basis of the present invention, was quite surprising even to the present inventors.

Consequently, up to now no suitable treatment for interstitial edema caused by hyaluronan accumulation has been available. The only treatment has been indirect and dependent on treatment of the inflammation. Then "nature" hopefully degrades the accumulated hyaluronan and hence reduces the edema.

The success of treatment of interstitial edema caused by inflammation with hyaluronidase depends on the fact that hyaluronan is not degraded in tissues which are not inflamed. Thus, we in our experiments have shown that the natural hyaluronan content of non-inflamed "control" kidneys is not disturbed.

### GENERAL DESCRIPTION OF THE INVENTION

The present inventors have been able to show that through an enzymatic digestion of hyaluronan accumulated in the connective tissue, excess water may be liberated and the tissue edema resulting from this increased content of hyaluronan thus may be alleviated. This to a man skilled in the art very surprising finding led the present inventors to develop a pharmaceutical based on hyaluronidase for the treatment of conditions of interstitial edema associated with an increased local content of hyaluronan in the connective tissue in connection with an organ grafting.

The object of the present invention consequently is the use of hyaluronidase in the manufacturing of a pharmaceutical as for the treatment of conditions of interstitial edema associated with an increased local content of hyaluronan in the connective tissue of a mammal of human or animal origin in connection with an organ grafting.

The invention provides a means for the treatment of conditions of interstitial edema associated with an increased local content of hyaluronan in the connective tissue of a mammal of human or animal origin in connection with an organ grafting, by systemic or local administration of a pharmaceutical comprising hyaluronidase.

The invention will be described in further detail herein below and is defined in the appended claims.

### DETAILED DESCRIPTION OF THE INVENTION

One preferred embodiment of the invention refers to the use of hyaluronidase in the manufacturing of a pharmaceutical for the treatment of interstitial edema in connection with an organ grafting in a mammal of animal or human origin. This treatment may be of a preventive or curative character. The organ may e.g. be a liver, a kidney or a heart, of a mammal of animal or human origin.

According to the invention, hyaluronidase is used in the manufacturing of a pharmaceutical to be administred systemically or locally.

Hyaluronidase should be incorporated in the pharmaceutical in such an amount as to give a daily dosage of from 1 IU to 100 000 IU by kg of the body weight, more preferably from 100 IU to 15 000 IU by kg of the body weight, or from 500 IU to 10 000 IU by kg of the body weight, by systemic or local administration of the pharmaceutical.

The pharmaceutical of the invention is prepared in a conventional way, known to the person skilled in the art. The locally applied pharmaceutical may in addition to hyaluronidase contain constituents of conventional organ preservation solutions, such as dextran, anions, cations, and osmotic molecules. Its systemic use form may contain stabilizing agents and saline.

The invention provides a means for the treatment of a mammal suffering from an interstitial edema occurring in connection with organ grafting and which is associated with increased local synthesis of hyaluronan, by systemic or local administration of a pharmaceutical comprising hyaluronidase.

according to the invention the active pharmaceutical is systemically or locally administrated to a mammal in an amount giving a daily dosage of from 1 to 100 000 IU of hyaluronidase/kg body weight of said mammal, more preferably from 100 IU to 15 000 IU by kg of the body weight, or from 500 IU to 10 000 IU by kg of the body weight, by local or systemic administation of the pharmaceutical.

The invention first was tested in a rat model of male Lewis rats weighing approximately 200g. A number of 10 rats were subjected to 60 min warm ischemia of the left kidney, to induce inflammatory edema and accumulation of hyaluronan. 5 of these animals were also treated with hyaluronidase i.v. at day 0, 1, and 2 and 2 hours before termination of the experiment. The five untreated animals developed water accumulation (edema) and hyaluronan accumulation in the left kidney. This phenomenon did not occur at all in the animals eadditionally treated with hyaluronidase i.v. There were no changes in water content or hyaluronan content of the right kidney as compared to measurements done in 5 naive rats.

Another study then was performed, as detailed herein below, by use of a method of heterotopic heart transplantation in rats:

Male PVG (RT1^{c}) and Wistar/Kyoto (RT1^{lv}) rats weighing 175-250 grams were used as donors and recipients, respectively. The animals were obtained from Møllegaard (Skensved, Denmark) and were allowed to settle for at least one week before operation, given free access to food and water. Surgery was performed under anesthesia with a mixture of chloral hydrate (180 mg/kg body weight), pentobarbital (40 mg/kg body weight) and magnesium sulphate (90 mg/kg body weight), administered intraperitoneally.

Heterotopic heart transplantations were performed using a non-suture technique; the aorta of the donor heart was anastomosed to the right common carotid artery of the recipient and the pulmonar artery to the right jugular vein. When the transplantation was finished a single dose of cefuroxim (Zinacef®, Glaxo, Greenford, UK), 20 mg/rat, was administered intramuscularly. Graft function was monitored by daily palpation and the hearts were regarded as rejected when no pulsations could be detected in the transplant any longer.

Immediately after reperfusion of the graft the recipient rat was given 20,000 U/kg body weight of sheep testes hyaluronidase, Type V (Sigma Chemical Co., St. Louis, MO, USA), administered intravenously. Intravenous doses of 20,000 U/kg body weight were then given once daily on days one, two, three and four and twice daily on day five; the last dose of 20,000 U/kg body weight was given on day six, two hours before harvesting. In addition, in this experimental group (n=11) the donor heart was flushed with hyaluronidase at the time of procurement; first the heart was flushed with 0.5 ml of FW-solution via the aorta and thereafter with another 0.5 ml of FW-solution containing 2,500 U of hyaluronidase. The rats in the control group (n=11) were left untreated until harvested on day six after transplantation. In this group the donor hearts were flushed with 1.0 ml of FW-solution without hyaluronidase.

As a separate experiment the effect of hyaluronidase-treatment on graft survival times was investigated. Two groups of rats were studied; untreated (n=10) and hyaluronidase-treated (n=11). This experiment was performed in the same way as described above, except that hyaluronidase was administered once daily as long as the grafts were functioning and that all animals were kept until the grafts were rejected.

At harvest one third of the transplanted heart was fixed in buffered 4% formalin, pH 7.3, with 1% cetylpyridiniumchloride, one third was snap frozen in cold isopentan and the remaining third was taken for determinations of the water- and HA-contents. For control purposes hearts were obtained from four untreated, non-transplanted rats.

Specimens were, immediately after harvesting, put on filter paper and weighed three minutes later (wet weight, w.w.). The specimens were lyophilized overnight and weighed again (dry weight, d.w.). After grinding, the HA was extracted from the tissues for 16 hours with 0.5 M NaCl. Following centrifugation for 15 minutes at 2,000 g the HA-content of the supernatants was analysed using a commercially available radiometric assay (Pharmacia Diagnostics, Uppsala, Sweden). The techniqe is based on the binding of HA to specific hyaluronic acid binding proteins (HABP). Briefly, 100 µl sample is incubated for 60 minutes at 4-7°C with 200 µl ¹²⁵I-labelled HABP. 100 µl HA-Sepharose is added and the incubation continued for 45 more minutes at the same temperature. Before centrifugation at 2,000 g for 10 minutes 2 ml washing solution is added.

After decantation the radioactivity in the pellet is measured in a gamma counter. A standard curve is constructed from samples with known amounts of HA. Double analyses were performed on each sample, with a variability of less then 10%. The relative water content, expressed as percent water of the total weight of the tissue, was calculated as 100 x (w.w. - d.w.)/w.w.

Data are given as mean values and standard errors of the mean (SEM). Comparison between groups were evaluated by Student's unpaired t-test for HA- and water-contents and by Mann-Whitney U-test for graft survival times. A p level of less than 0.05 was regarded as statistically significant.
The results are listed in the table herein below.

**TABLE**

| Extracted hyaluronan amounts and relative water contents (mean±SEM) of allogeneic heart grafts on day 6 after transplantation and the effects of daily intravenous hyaluronidase administration. | | | |
|---|---|---|---|
| | n | Hyaluronan (µg/g d.w.) | Water (%) |
| Allogeneic transplantation | 11 | 789±47 | 81.8±0.3 |
| Allogeneic transplantation and hyaluronidase | 11 | 494±43*** | 80.6±0.3** |
| Controls | 4 | 206±18 | 76.3±0.3 |

| | | | |
|---|---|---|---|
| The extraction of hyaluronan was performed from freeze-dried heart tissue with 0.5 M NaCl. ** p<0.01, *** p<0.001. Statistical differences between the hyaluronan and water contents of the heart grafts with and without hyaluronidase were tested using the Mann-Whitney U-test. | | | |

In order to locate the distribution of HA immunohistochemical stainings were performed. For this purpose an avidin-enzyme, biotin-protein system was used mainly as described earlier. In brief, paraffin-embedded four µm thick sections were incubated with bovine serum albumin (10 mg/ml, Fraction V, Sigma Chemical Co, St. Louis, MO, USA) to block non-specific binding sites and, thereafter, in 3% H₂O₂ in PBS to inhibit endogenous peroxidase. After incubation for 2 h with HABP the sections were incubated with ABC Vectastain Reagent (Vector Laboratories, Burlingame, CA, USA) for 1 h. Finally, H₂O₂ as substrate and 3-amino-9-etyl-carbazole (AEC) as electron donor were added, whereafter the specimens were counterstained with Mayer's haematoxylin. Control sections were incubated for 2 h with Streptomyces hyaluronidase. All slides were evaluated blindly.

Cardiac grafts fixed in formalin were embedded in paraffin, cut into 4 µm thick sections and stained with eosin and Mayer's haematoxylin. All slides were evaluated blindly.

In the tissue sections of the cardiac grafts six days after allogeneic transplantation, pronounced cell infiltration was seen in the widened interstitial space. Accumulation of hyaluronan was seen in the cell-rich widened interstitium. After daily intravenous administration of hyaluronidase the interstitial space was reduced and cell-poor and the staining for hyaluronan was quite discrete.

Hyaluronidase treatment in the present rat model not only reduced edema formation but also significantly improved the graft survival time. Thus, in untreated rats graft rejection occurred after 8.3±0.3 days whereas daily intravenous treatment with hyaluronidase prolonged graft survival times with 1.5 days (mean survival time 9.7±0.5 days; p<0.01). It is noteable that such a striking effect was obtained in this totally non-immunosuppressed system where rejection development is such a fast process.

## Claims

1. The use of hyaluronidase in the manufacturing of a drug for the treatment of conditions of interstitial edema in connection with organ grafting associated with an increased local content of hyaluronan in the connective tissue of a mammal of human or animal origin.

2. The use according to claim 1 in the manufacturing of a drug comprising hyaluronidase in an amount giving a daily dosage of from 1 to 100 000 IU/kg body weight when it is to be given locally or systemically to said mammal.

3. The use according to claim 2 wherein said daily dosage is from 100 to 15 000 IU/kg body weight.

4. The use according to claim 3 wherein said daily dosage is from 500 to 10 000 IU/kg body weight.

5. The use according to any of the above claims in the manufacturing of a drug to be given preventively or curatively.

## Patentansprüche

1. Verwendung von Hyaluronidase beim Herstellen eines Arzneistoffes zur Behandlung von Zuständen interstitieller Ödeme in Verbindung mit Organtransplantation, verbunden mit einem erhöhten lokalen Gehalt von Hyaluronan im Bindegewebe eines Säugers menschlicher oder tierischer Herkunft.

2. Verwendung nach Anspruch 1 bei der Herstellung eines Arzneistoffes, der Hyaluronidase in einer Menge umfaßt, die eine tägliche Dosis von 1 bis 100.000 I.E./kg Körpergewicht ergibt, wenn er dem Säuger örtlich oder systemisch verabreicht werden soll.

3. Verwendung nach Anspruch 2, wobei die tägliche Dosis 100 bis 15.000 I.E./kg Körpergewicht beträgt.

4. Verwendung nach Anspruch 3, wobei die tägliche Dosis 500 bis 10.000 I.E./kg Körpergewicht beträgt.

5. Verwendung nach einem der obigen Ansprüche bei der Herstellung eines Arzneistoffes, der präventiv oder kurativ verabreicht werden soll.

## Revendications

1. Utilisation de la hyaluronidase dans la fabrication d'un médicament pour le traitement d'états d'oedème interstitiel en rapport avec une greffe d'organe associée à une augmentation de la teneur locale en hyaluronane dans le tissu conjonctif d'un mammifère d'origine humaine ou animale.

2. Utilisation selon la revendication 1 dans la fabrication d'un médicament comprenant de la hyaluronidase dans une quantité donnant une posologie quotidienne allant de 1 à 100 000 UI/kg de poids corporel lorsqu'il doit être administré par voie locale ou générale au dit mammifère.

3. Utilisation selon la revendication 2, dans laquelle ladite posologie quotidienne est de 100 à 15 000 UI/kg de poids corporel.

4. Utilisation selon la revendication 3, dans laquelle ladite posologie quotidienne est de 500 à 10 000 UI/kg de poids corporel.

5. Utilisation selon l'une quelconque des revendications précédentes dans la fabrication d'un médicament à administrer à titre préventif ou curatif.
